## Europäisches Patentamt
## European Patent Office
## Office européen des brevets

(19)

(11) Veröffentlichungsnummer: **0 123 042**
**B1**

(12) **EUROPÄISCHE PATENTSCHRIFT**

(45) Veröffentlichungstag der Patentschrift:
**23.07.86**

(21) Anmeldenummer: **84101702.3**

(22) Anmeldetag: **18.02.84**

(51) Int. Cl.⁴: **C 07 C 49/707**, C 07 C 45/27,
C 07 C 45/43

(54) **Verfahren zur Herstellung von Quadratsäure.**

(30) Priorität: **21.04.83 DE 3314431**

(43) Veröffentlichungstag der Anmeldung:
**31.10.84 Patentblatt 84/44**

(45) Bekanntmachung des Hinweises auf die Patenterteilung:
**23.07.86 Patentblatt 86/30**

(84) Benannte Vertragsstaaten:
**CH DE FR GB IT LI**

(56) Entgegenhaltungen:
**DE-A-2 824 558**
**FR-A-1 404 928**
**FR-A-2 352 778**

(73) Patentinhaber: **HÜLS AKTIENGESELLSCHAFT, -
RSP Patente / PB 15 - Postfach 13 20, D-4370
Marl 1 (DE)**

(72) Erfinder: **Rombusch, Konrad, Dr., Flämingstrasse
5, D-4370 Marl (DE)**
Erfinder: **Maahs, Günther, Dr., Oderbruchstrasse
19, D-4370 Marl (DE)**

EP 0 123 042 B1

## Beschreibung

Quadratsäure (1, 2-Dihydroxycyclobutendion-3, 4) ist ein wertvolles Zwischenprodukt zur Herstellung von Stabilisatoren, Farbstoffen, Bakteriziden und Fungiziden.

Es sind bereits eine Reihe von Verfahren zu seiner Herstellung bekannt, wie in der DE-PS 26 23 836 ausführlich beschrieben wird. Nach dem in der genannten Patentschrift beanspruchten Verfahren erfolgt die Herstellung von Quadratsäure durch Umsetzung von Hexachlorcyclobuten mit 70 bis 96 gewichtsprozentiger Schwefelsäure bei Temperaturen zwischen 80 und 150 °C. Die besten Ergebnisse werden durch Umsetzung von Hexachlorcyclobuten mit 90 gewichtsprozentiger Schwefelsäure bei 120 °C erhalten. Dabei werden zur Umsetzung des Hexachlorcyclobutens zur Quadratsäure 4 bis 10 Stunden und zum Nachreagieren und Austreiben des gelöst gebliebenen Anteils des entstandenen Chlorwasserstoffs im allgemeinen weitere 3 Stunden benötigt.

Durch die Erhöhung der Reaktionstemperaturen von 120 °C auf beispielsweise 140 °C kann man zwar erwartungsgemäß die benötigte Reaktionszeit senken, doch bilden sich dann Verkohlungsprodukte, die die Quadratsäure verfärben (Beispiel 7).

Weicht die Schwefelsäurekonzentration deutlich vom optimalen Wert von 90 Gewichtsprozent sowohl nach unten als auch nach oben ab, steigt die aufzuwendende Reaktionszeit an. Dies führt dazu, daß unterhalb einer Schwefelsäurekonzentration von 70 Gewichtsprozent selbst bei Anwendung von höheren Temperaturen und von Druck nur noch geringe Mengen Quadratsäure gebildet werden und ebenso, daß oberhalb einer Schwefelsäurekonzentration von 96 Gewichtsprozent infolge des geringen Wasserangebots ebenfalls praktisch keine Hydrolyse mehr stattfindet (Spalte 2, Zeilen 12 bis 24).

Es wird in der genannten Patentschrift daher empfohlen, während der Reaktion Wasser (Beispiel 5) oder verdünnte Schwefelsäure (Beispiel 3) hinzuzugeben oder von Anfang an ausreichende Mengen 90%iger Schwefelsäure einzusetzen, um die optimale Wasserkonzentration einzuhalten. Die Beispiele belegen, daß die Schwefelsäurekonzentration während der gesamten Reaktion stets deutlich unter 96 Gewichtsprozent bleibt.

Zwar kann man nach dem beschriebenen Verfahren auch Mischungen von Hexachlorcyclobuten mit Hexachlorbutadien umsetzen, doch zeigt das Beispiel 4, daß eine Mischung von je 50% Hexachlorcyclobuten und Hexachlorbutadien bei 120 °C erst nach 15 Stunden eine gegenüber dem Optimum um 20% verringerte Ausbeute ergibt. Solche Ergebnisse sind für technische Verfahren nicht akzeptabel.

Es kommt hinzu, daß das Reaktionsprodukt nach einer so langen Reaktionszeit gefärbt ist.

Mischungen, deren Hexachlorcyclobutenanteil noch geringer ist, sind praktisch kaum noch verwendbar.

Andererseits ist man aus wirtschaftlichen und technischen Überlegungen sehr daran interessiert, Mischungen mit einem möglichst niedrigen Anteil an Hexachlorcyclobuten einsetzen zu können. Bekanntlich sind solche Mischungen wegen des geringeren Rektifizieraufwandes kostengünstiger herzustellen (vgl. DE-PS 26 18 557). Derartig niedrigprozentige Lösungen haben ferner in der Handhabung den Vorteil, daß der Kristallisationspunkt des cyclischen Isomeren so tief liegt, daß es in der Lösung bei Zimmertemperatur nicht ausfallen kann.

Die DE-PS 28 24 558 beschreibt ein weiteres Verfahren zur Herstellung von Quadratsäure, wobei man Hexachlorcyclobuten oder dessen Gemisch mit Hexachlorbutadien zunächst mit Oleum auf 60 bis 120 °C erhitzt und anschließend in einem zweiten Verfahrensschritt im Reaktionsgemisch das organische Zwischenprodukt zu Quadratsäure und die gebildete Chlorsulfonsäure zu Schwefelsäure hydrolysiert.

Abgesehen davon, daß man den Einsatz von Oleum aus Kostengründen und wegen seiner aggressiven und toxischen Wirkung möglichst zu vermeiden versucht, hat dieses Verfahren weitere Nachteile. Es sind besondere Vorsichtsmaßnahmen erforderlich, um die bei der Reaktion gebildete Chlorsulfonsäure zu hydrolysieren. Unerwartet und gravierend ist der Umstand, daß es im Laufe der Reaktion zur Bildung extrem tränenreizender Produkte kommt. Unter diesen Umständen stößt eine Realisierung des Verfahrens in der Praxis auf erhebliche technische und toxikologische Bedenken.

Der DE-PS 28 24 558 ist keine Anregung zu entnehmen, mit Oleum niedriger $SO_3$-Konzentration zu arbeiten, da dann die Menge der Schwefelsäure deutlich erhöht werden müßte.

Es stellte sich daher die Aufgabe, ein Verfahren zur Herstellung von Quadratsäure zu entwickeln,

1. das den Einsatz von Gemischen von Hexachlorcyclobuten und Hexachlor-1,3-butadien zuläßt, insbesondere solchen, in denen der Anteil des cyclischen Isomeren unter 55 % liegt,

2. dessen Reaktionszeiten kürzer sind, als in der DE-PS 26 23 836 angegeben,

3. dessen Temperatur so niedrig eingestellt werden kann, daß es nicht zur Bildung gefärbter Endprodukte kommt,

4. das die Rückgewinnung der eingesetzten Schwefelsäure zuläßt und

5. das ohne toxikologische Bedenken im technischen Maßstab durchgeführt werden kann.

Es wurde jetzt gefunden, daß man diese Ziele erreichen kann, wenn man die Umsetzung von Hexachlorcyclobuten bzw. seinen Gemischen mit Hexachlor-1, 3-butadien in Gegenwart einer Schwefelsäure durchführt, die einen Wassergehalt unter 4 Gewichtsprozent aufweist und man andererseits dafür sorgt, daß zumindest die stöchiometrisch erforderliche Menge Wasser zur Verfügung steht.

Grundlage für dieses Verfahren ist der überraschende Befund, daß die Reaktionsgeschwindigkeit im beanspruchten Bereich mit sinkender Wasserkonzentration nicht abnimmt, sondern im Gegenteil stark ansteigt, sofern nur die stöchiometrisch erforderliche Menge vorhanden ist. Diese Erkenntnis widerspricht nicht nur den Angaben in der DE-PS 26 23 836, sie steht auch im Gegensatz zu der üblichen Annahme einer Proportionalität von Reaktionsgeschwindigkeit und Konzentration der Reaktionspartner.

Bei dem Verfahren gemäß der vorliegenden Erfindung geht man von mehr als 96%iger, vorzugsweise mehr als 96,5%iger oder noch besser mehr als 98 %iger Schwefelsäure aus und setzt im Laufe der Reaktion Wasser oder verdünnte Schwefelsäure zu. Man kann auch von 100 %iger Schwefelsäure ausgehen und erst im Laufe der Reaktion, zweckmäßigerweise nach Beendigung der Abspaltung von ca. 2 Mol Chlorwasserstoff pro Mol Hexachlorcyclobuten, Wasser oder verdünnte Schwefelsäure zusetzen.

Die Reaktion wird bei Temperaturen zwischen 80 und 150 °C, insbesondere bei 110 bis 135 °C, durchgeführt. Besonders vorteilhaft ist der Bereich 120 bis 125 °C, weil die Reaktionsgeschwindigkeit hier groß genug und die Bildung von Verkohlungsprodukten noch sehr gering sind.

Die Zugabe von Hexachlorcyclobuten zur Schwefelsäure kann entweder vollständig zu Beginn der Reaktion oder portionsweise zu Beginn und im weiteren Verlauf der Reaktion, z.B. nach Abspaltung von bestimmten Prozentsätzen des abzuspaltenden Chlorwasserstoffs, erfolgen.

Die Menge der eingesetzten Schwefelsäure hängt, wie bereits ausgeführt, von der jeweils gewählten Verfahrensvariante ab. Am günstigsten ist es, pro Mol Hexachlorcyclobuten 6 bis 14 Mole Schwefelsäure einzusetzen, von denen sich mehr als 90% wiedergewinnen lassen. Soll die Schwefelsäure nicht wieder verwendet werden, setzt man der Reaktionsmischung solange Wasser zu, bis die Schwefelsäure einen Gehalt von 65 bis 75 % erreicht hat, und fällt die Quadratsäure aus.

Wird die Schwefelsäure dagegen für den folgenden Reaktionsansatz benötigt, kühlt man das Reaktionsgemisch nach Beendigung der Chlorwasserstoffentwicklung auf 20°C, vorzugsweise 10°C, ab. Man kann das Reaktionsprodukt abfiltrieren, im Filtrat gegebenenfalls Hexachlorbutadien abtrennen und die zurückbleibende Schwefelsäure unmittelbar der nächsten Reaktion zuführen. Die außerordentlich geringen Verluste an Schwefelsäure, die auf diese Weise auftreten, können durch Zugabe frischer Säure leicht ausgeglichen werden.

Die zur Gehaltsbestimmung der Quadratsäure verwendete 0,1 n KMnO$_4$-Lösung wurde an einer viermal mit Wasser umkristallisierten Probe geeicht. Diese Probe hatte potentiometrisch einen Gehalt an Quadratsäure von 99,75 %, einen Wassergehalt von 0,06%, einen Cl-Gehalt von 0, 003 % und einen S-Gehalt von 0,0003%.

## Beispiel 1

In einem 1 l-Glaskolben wurde unter Rühren (Flügel-Rührer, 350 U/min) das Gemisch aus 129, 6 g (0,497 Mol) Hexachlorcyclobuten, 132,2 g (0,507 Mol) Hexachlorbutadien und 663 g 96,2 gewichtsprozentige Schwefelsäure (6, 56 Mol) auf 122 °C erhitzt. Der abgespaltene Chlorwasserstoff wurde in einer Vorlage, gefüllt mit 15%iger Natronlauge, absorbiert und die absorbierte Menge titrimetrisch bestimmt. Nach Absorption von jeweils 0,05 bis 0,25 Mol Chlorwasserstoff wurde die durch die Hydrolysereaktion verbrauchte Wassermenge, d. h. 35,8 g Wasser so zudosiert, daß die Wasserkonzentration in der Schwefelsäure 2,0 bis 3, 8 Gewichtsprozent, bezogen auf die Summe Schwefelsäure und Wasser, betrug. Nach 3,5 Stunden waren 2,7 Mol Chlorwasserstoff abgespalten, daveon 25% nach 0,48 Stunden, 50% nach 0,87 Stunden, 75% nach 1,32 Stunden und 95% nach 2,54 Stunden. Nach Beendigung der Reaktion wurden zur Reaktionslösung 330 g Wasser zugefügt und abgekühlt. Die ausgefallene Quadratsäure wurde abgesaugt, dreimal mit je 15 cm³ Wasser, anschließend mit 200 cm³ Petrolether gewaschen und dann getrocknet. Man erhielt 53,9 g Rohprodukt. Die Kaliumpermanganat-Titration ergab eine Ausbeute von 88,3%.

## Beispiel 2

In einem 1 l-Glaskolben wurde unter Rühren (Flügel-Rührer, 350 U/min) das Gemisch aus 130,6 g (0,501 Mol) Hexachlorcyclobuten, 130,4 g (0,50 Mol) Hexachlorbutadien und 650 g 99,8 gewichtsprozentiger Schwefelsäure (6,61 Mol) auf 125 °C erhitzt. Der abgespaltene Chlorwasserstoff wurde in einer Vorlage, gefüllt mit 15%iger Natronlauge, absorbiert und die absorbierte Menge titrimetrisch bestimmt. Nach Absorption von jeweils 0,05 bis 0,25 Mol Chlorwasserstoff wurde die durch die Hydrolysereaktion verbrauchte Wassermenge, d. h. 36 g Wasser so zudosiert, daß die Wasserkonzentration in der Schwefelsäure 0,2 bis 0,4 Gewichtsprozent, bezogen auf die Summe Schwefelsäure und Wasser, betrug. Nach Beendigung der Reaktion wurden zum Reaktionsgemisch 345 g Wasser zugefügt. Nach dem Abkühlen wurde die ausgefallene Quadratsäure abgesaugt, dreimal mit je 15 cm³ Wasser, anschließend mit 200 cm Petrolether gewaschen und dann getrocknet. Man erhielt 51,4 g Rohprodukt. Die Kaliumpermanganat-Titration ergab eine Ausbeute von 83,3%.

**Beispiel 3**

In einem 1 l-Glaskolben wurde unter Rühren (Flügel-Rührer, 350 U/min) das Gemisch aus 104,3 g (0,400 Mol) Hexachlorcyclobuten, 104,6 g (0,401 Mol) Hexachlorbutadien und 533,8 g 97,0 gewichtsprozentiger Schwefelsäure (5,28 Mol) auf 135 °C erhitzt. Der abgespaltene Chlorwasserstoff wurde in einer Vorlage, gefüllt mit 15 %iger Natronlauge, absorbiert und die absorbierte Menge titrimetrisch bestimmt. Nach Absorption von jeweils 0,05 bis 0,25 Mol Chlorwasserstoff wurde die durch die Hydrolysereaktion verbrauchte Wassermenge, d. h. 2,9 g Wasser so zudosiert, daß die Wasserkonzentration in der Schwefelsäure 1,7 bis 3,0 Gewichtsprozent, bezogen auf die Summe Schwefelsäure und Wasser, betrug. Nach Beendigung der Reaktion wurden zum Reaktionsgemisch 260 g Wasser zugefügt. Nach dem Abkühlen wurde die ausgefallene Quadratsäure abgesaugt, dreimal mit je 15 cm³ Wasser, anschließend mit 200 cm³ Petrolether gewaschen und dann getrocknet. Man erhielt 41,0 g Rohprodukt. Die Kaliumpermanganat-Titration ergab eine Ausbeute von 86,2 %.

**Beispiel 4**

In einem 1 l-Glaskolben wurde unter Rühren (Flügel-Rührer, 350 U/min) das Gemisch aus 32,7 g (0,125 Mol) Hexachlorcyclobuten, 33,3 g (0,128 Mol) Hexachlorbutadien und 664,2 g 97,0 gewichtsprozentiger Schwefelsäure (6,57 Mol) auf 120 °C erhitzt. Der abgespaltene Chlorwasserstoff wurde in einer Vorlage, gefüllt mit 15%iger Natronlauge, absorbiert und die absorbierte Menge titrimetrisch bestimmt. Nach Absorption von jeweils 0,05 bis 0,25 Mol Chlorwasserstoff wurde die durch die Hydrolysereaktion verbrauchte Wassermenge, d. h. 9 g Wasser so zudosiert, daß die Wasserkonzentration in der Schwefelsäure 2,0 bis 3,5 Gewichtsprozent, bezogen auf die Summe Schwefelsäure und Wasser, betrug. Nach Abspaltung von 0,06 Mol HCl (das war nach 0,1 Stunden) wurde ein Gemisch aus 32,4 g (0,124 Mol) Hexachlorcyclobuten und 32,9 g (0,126 Mol) Hexachlorbutadien zugesetzt, nach Abspaltung von weiteren 0,86 Mol HCl (das war nach 0,77 Stunden) ein Gemisch aus 64,8 g (0,249 Mol) Hexachlorcyclobuten und 65,9 g (0,253 Mol) Hexachlorbutadien und nach Abspaltung von weiteren 1,19 Mol HCl (das war nach 1,75 Stunden) ein Gemisch aus 77,9 g (0,299 Mol) Hexachlorcyclobuten und 79,1 g (0,303 Mol) Hexachlorbutadien zugegeben. Nach Beendigung der Reaktion wurden zum Reaktionsgemisch 330 g Wasser zugefügt. Nach dem Abkühlen wurde die ausgefallene Quadratsäure abgesaugt, dreimal mit je 15 cm³ Wasser, anschließend mit 200 cm³ Petrolether gewaschen und dann getrocknet. Man erhielt 84,2 g Rohprodukt. Die Kaliumpermanganat-Titration ergab eine Ausbeute von 87,1 %.

**Beispiel 5**

In einem 1 l-Glaskolben wurde unter Rühren (Flügel-Rührer, 350 U/min) das Gemisch aus 43,3 g (0,166 Mol) Hexachlorcyclobuten, 0,7 g (0,003 Mol) Hexachlorbutadien und 215 g 100 gewichtsprozentiger Schwefelsäure (2,19 Mol) 0,75 Stunden auf 125 °C erhitzt. Der abgespaltene Chlorwasserstoff wurde in einer Vorlage, gefüllt mit 15%iger Natronlauge, absorbiert und die absorbierte Menge titrimetrisch bestimmt. Anschließend wurden innerhalb 1 Stunde bei 115 °C 21,0 g Wasser in die Reaktionsmischung getropft und 1 Stunde bei derselben Temperatur nachgerührt. Dabei wurden weitere 0,56 Mol Chlorwasserstoff abgespalten. Zur Reaktionslösung wurden 1 10 g Wasser zugefügt. Nach dem Abkühlen wurde die ausgefallene Quadratsäure abgesaugt, dreimal mit je 15 cm Wasser, anschließend mit 200 cm³ Petrolether gewaschen und dann getrocknet. Man erhielt 18,2 g Rohprodukt. Die Kaliumpermanganat-Titration ergab eine Ausbeute von 91,6%.

**Beispiel 6**

In einem 1 l-Glaskolben wurde unter Rühren das Gemisch aus 130,0 g (0,50 Mol) Hexachlorcyclobuten, 130,0 g (0,50 Mol) Hexachlorbutadien und 414,1 g 97,1 gewichtsprozentiger Schwefelsäure (4,1 Mol) auf 120 °C erhitzt. Der abgespaltene Chlorwasserstoff wurde in einer Vorlage, gefüllt mit 15 %iger Natronlauge, absorbiert und die absorbierte Menge titrimetrisch bestimmt. Nach Absorption von jeweils 0,05 bis 0,25 Mol Chlorwasserstoff wurde die durch die Hydrolysereaktion verbrauchte Wassermenge so zudosiert, daß die Wasserkonzentration in der Schwefelsäure 2,0 bis 3,0 Gewichtsprozent, bezogen auf die Summe Schwefelsäure und Wasser, betrug. Nach dem Abkühlen auf 0 °C wurde die ausgefallene Quadratsäure abgesaugt, dreimal mit je 15 cm³ Wasser, anschließend mit 200 cm³ Petrolether gewaschen und dann getrocknet. Man isolierte 44,2 g Produkt. Die Kaliumpermanganat-Titration ergab eine Ausbeute von 72,4%.

Die organische Phase des zurückbleibenden Filtrats wurde abgetrennt und der Verlust an Säure durch Zugabe von 21 g 97 %iger Schwefelsäure ausgeglichen. Daraufhin wurde die Schwefelsäure ein zweites Mal in einem 1 l-Glaskolben unter Rühren zusammen mit einem Gemisch aus 130,0 g (0,50 Mol) Hexachlorcyclobuten, 130,0 g (0,50 Mol) Hexachlorbutadien auf 120 °C erhitzt. Der abgespaltene Chlorwasserstoff wurde in einer Vorlage, gefüllt mit 15%iger Natronlauge, absorbiert und die absorbierte Menge titrimetrisch bestimmt. Nach Absorption von jeweils 0,05 bis 0,25 Mol Chlorwasserstoff wurde die durch die Hydrolysereaktion verbrauchte Wassermenge so zudosiert, daß die Wasserkonzentration in der Schwefelsäure 2,0 bis 3,0 Gewichtsprozent, bezogen auf die Summe Schwefelsäure und Wasser, betrug. Nach dem Abkühlen wurde die ausgefallene Quadratsäure abgesaugt, dreimal mit je 15 ml Wasser, anschließend mit 200 ml Petrolether gewaschen und dann getrocknet. Die Kaliumpermanganat-Titration ergab eine Ausbeute von 84,8 %. Das Filtrat wurde in gleicher Weise wie vorstehend beschrieben, aufgearbeitet und noch zweimal wieder eingesetzt. Dann wurde es verworfen.

**Beispiel 7**

In einem 1 l-Glaskolben wurde unter Hühren (Flügel-Rührer, 350 U/min) das Gemisch aus 104,3 g (0,400 Mol) Hexachlorcyclobuten, 243,3 g (0,933 Mol) Hexachlorbutadien und 533,8 g 97,0 gewichtsprozentiger Schwefelsäure (5,28 Mol) auf 123 °C erhitzt. Der abgespaltene Chlorwasserstoff wurde in einer Vorlage, gefüllt mit 15%iger Natronlauge, absorbiert und die absorbierte Menge titrimetrisch bestimmt. Nach Absorption von jeweils 0,05 bis 0,25 Mol Chlorwasserstoff wurde die durch die Hydrolysereaktion verbrauchte Wassermenge so zudosiert, daß die Wasserkonzentration in der Schwefelsäure 2,0 bis 3,0 Gewichtsprozent, bezogen auf die Summe Schwefelsäure und Wasser, betrug. Nach Beendigung der Reaktion wurden zum Reaktionsgemisch 230 g Wasser zugefügt. Nach dem Abkühlen wurde die ausgefallene Quadratsäure abgesaugt, dreimal mit je 15 cm$^3$ Wasser, anschließend mit 200 cm$^3$ Petrolether gewaschen und dann getrocknet. Man erhielt 39,8 g Rohprodukt. Die Kaliumpermanganat-Titration ergab eine Ausbeute von 84,1 %.

**Beispiel 8**

In einem 1 l-Glaskolben wurde unter Rühren (Flügel-Rührer, 350 U/min) das Gemisch aus 104,3 g (0,40 Mol) Hexachlorcyclobuten, 104,3 g (0,40 Mol) Hexachlorbutadien und 266,9 g 97,0 gewichtsprozentiger Schwefelsäure (2,64 Mol) auf 125 °C erhitzt. Der abgespaltene Chlorwasserstoff wurde in einer Vorlage, gefüllt mit 15 %iger Natronlauge, absorbiert und die absorbierte Menge titrimetrisch bestimmt. Nach Absorption von jeweils 0,1 bis 0,5 Mol Chlorwasserstoff pro Mol eingesetztem Hexachlorcyclobuten wurde die durch die Hydrolysereaktion verbrauchte Wassermenge, d. h. 4 Mol Wasser auf 6 Mol Chlorwasserstoff, so zudosiert, daß die Wasserkonzentration in der Schwefelsäure 2,1 bis 3,2 Gewichtsprozent, bezogen auf die Summe Schwefelsäure und Wasser, betrug. Nach 6,0 Stunden waren 2,10 Mol Chlorwasserstoff abgespalten, davon 25 % nach 0,63 Stunden, 50 % nach 1,15 Stunden, 75 % nach 2,03 Stunden und 95% nach 3,80 Stunden. Nach Beendigung der Reaktion wurden zum Reaktionsgemisch 130 g Wasser zugefügt. Nach dem Abkühlen wurde die ausgefallene Quadratsäure abgesaugt, dreimal mit je 15 cm$^3$ Wasser, mit 250 cm$^3$ Petrolether gewaschen und dann getrocknet. Man erhielt 36,8 g Rohprodukt. Die Kaliumpermanganat-Titration ergab eine Ausbeute von 84, 1 %.

**Beispiel 9**

In einem 1 l-Glaskolben wurde unter Rühren (Flügel-Rührer, 350 U/ min) das Gemisch aus 129,7 g (0,497 Mol) Hexachlorcyclobuten, 3,1 g (0,012 Mol) Hexachlorbutadien und 667,3 g 97,0 gewichtsprozentiger Schwefelsäure (6,6 Mol) auf 120 °C erhitzt. Der abgespaltene Chlorwasserstoff wurde in einer Vorlage, gefüllt mit 15 %iger Natronlauge, absorbiert und die absorbierte Menge titrimetrisch bestimmt. Nach Absorption von jeweils 0,1 bis 0,5 Mol Chlorwasserstoff pro Mol eingesetztem Hexachlorcyclobuten wurde die durch die Hydrolysereaktion verbrauchte Wassermenge so zudosiert, daß die Wasserkonzentration in der Schwefelsäure 2,0 bis 3,3 Gewichtsprozent, bezogen auf die Summe Schwefelsäure und Wasser, betrug. Nach 2,25 Stunden waren 2,70 Mol Chlorwasserstoff abgespalten, 25% davon nach 0,28 Stunden, 50% nach 0,49 Stunden, 75 % nach 0,69 Stunden und 95 % nach 0,95 Stunden. Nach Beendigung der Reaktion wurden zum Reaktionsgemisch 320 g Wasser zugefügt. Nach dem Abkühlen wurde die ausgefallene Quadratäsure abgesaugt, dreimal mit je 15 cm$^3$ Wasser, mit 200 cm$^3$ Petrolether gewaschen und dann getrocknet. Man erhielt 52,3 g Rohprodukt. Die Kaliumpermanganat-Titration ergab eine Ausbeute von 87,7%.

**Beispiel 10**

In einem 1 l-Glaskolben wurde unter Rühren (Propeller-Rührer, 1 200 U/min) das Gemisch aus 129,6 g (0,497 Mol) Hexachlorcyclobuten, 132,2 g (0,507 Mol) Hexachlorbutadien und 670,2 g 96,0 gewichtsprozentiger Schwefelsäure (6,56 Mol) auf 110 °C erhitzt. Der abgespaltene Chlorwasserstoff wurde in einer Vorlage, gefüllt mit 15%iger Natronlauge, absorbiert und die absorbierte Menge titrimetrisch bestimmt. Nach Absorption von jeweils 0,1 bis 0,5 Mol Chlorwasserstoff pro Mol eingesetztem Hexachlorcyclobuten wurde die durch die Hydrolysereaktion verbrauchte Wassermenge so zudosiert, daß die Wasserkonzentration in der Schwefelsäure 2,0 bis 4,0 Gewichtsprozent, bezogen auf die Summe Schwefelsäure und Wasser, betrug. Nach 8,5 Stunden waren 2,59 Mol Chlorwasserstoff abgespalten, davon 25 % nach 1,58 Stunden, 50% nach 2,39 Stunden, 75% nach 3,78 Stunden und 95 % nach 6,18 Stunden. Nach Beendigung der Reaktion wurden zum Reaktionsgemisch 330 g Wasser zugefügt. Nach dem Abkühlen wurde die ausgefallene Quadratsäure abgesaugt, dreimal mit je 15 cm³ Wasser, mit 200 cm³ Petrolether gewaschen und dann getrocknet. Man erhielt 49,8 g Rohprodukt. Die Kaliumpermanganat-Titration ergab eine Ausbeute von 83,7 %.

### Beispiel 11

In einem 1 1-Glaskolben wurde unter Rühren (Flügel-Rührer, 350 U/ min) das Gemisch aus 149,9 g (0,575 Mol) Hexachlorcyclobuten, 149,2 g (0,572 Mol) Hexachlorbutadien und 743 g 100 gewichtsprozentiger Schwefelsäure (7,58 Mol) 1,5 Stunden auf 120 bis 125°C erhitzt. Der abgespaltene Chlorwasserstoff wurde in einer Vorlage, gefüllt mit 15%iger Natronlauge, absorbiert und die absorbierte Menge titrimetrisch bestimmt: 1,04 Mol. Anschließend wurden innerhalb 1 Stunde bei 120 bis 125 °C 72,4 g Wasser in die Reaktionsmischung getropft und 0,75 Stunden bei derselben Temperatur nachgerührt.

Dabei wurden weitere 2,21 Mol Chlorwasserstoff abgespalten. Zur Reaktionslösung wurden 370 g Wasser zugefügt. Nach dem Abkühlen wurde die ausgefallene Quadratsäure abgesaugt, dreimal mit je 15 cm³ Wasser, mit 200 cm³ Petrolether gewaschen und dann getrocknet. Man erhielt 59,3 g Rohprodukt. Die Kaliumpermanganat-Titration ergab eine Ausbeute von 83,7 %.

### Patentansprüche

1. Verfahren zur Herstellung von Quadratsäure aus Hexachlorcyclobuten oder dessen Gemischen mit Hexachlor-1,3-butadien und Schwefelsäure bei einer Temperatur von 80 bis 150 °C,
dadurch gekennzeichnet,
daß einerseits der Wassergehalt der Schwefelsäure während der Reaktion stets unter 4 % bleibt und andererseits wenigstens die für die Bildung von Quadratsäure stöchiometrisch erforderliche Menge Wasser im Laufe der Reaktion direkt oder in Form verdünnter Schwefelsäure zugesetzt wird.

2. Verfahren nach Anspruch 1,
dadurch gekennzeichnet,
daß während der Reaktion eine wasserhaltige Schwefelsäure vorliegt, deren Wassergehalt stets unter 3,5%, vorzugsweise unter 2 %, bleibt.

3. Verfahren nach den Ansprüchen 1 und 2,
dadurch gekennzeichnet,
daß man von einer wasserfreien Schwefelsäure ausgeht und man den Gehalt an Wasser nach Beendigung der Chlorwasserstoffentwicklung auf einen Wert zwischen 0,05 und 3,9 Gewichtsprozent einstellt.

4. Verfahren nach den Ansprüchen 1 bis 3,
dadurch gekennzeichnet,
daß man ein Gemisch aus Hexachlorcyclobuten und Hexachlorbutadien einsetzt, das 55% und weniger Hexachlorcyclobuten enthält.

### Claims

1. A process for the production of squaric acid from hexachlorocyclobutene or a mixture thereof with hexa-chloro-1,3-butadiene and sulphuric acid at a temperature of 80 to 150°C, characterised in that on the one hand the water content of the sulphuric acid remains always below 4% during the reaction and on the other hand at least the stoichiometric amount of water for the formation of squaric acid is introduced in the course of the reaction either as such or in the form of dilute sulphuric acid.

2. A process according to claim 1, characterised in that during the reaction a water-containing sulphuric acid is present, the water content of which always remains below 3.5%, preferably below 2%.

3. A process according to claim 1 or 2, characterised in that a water-free sulphuric acid is used initially and the content of water is increased to a value from 0.05 to 3.9% by weight after the end of the hydrogen chloride removal.

4. A process according to any of claims 1 to 3, characterised in that a mixture of hexachlorocyclobutene and hexachlorobutadiene is used which contains 55 % or less hexachlorocyclobutene.

### Revendications

1. Procédé pour la préparation d'acide quadratique en partant d'hexachloro-cyclobutène ou de mélanges de celui-ci avec l'hexachloro-1,3-butadiène et d'acide sulfurique à une température de 80 à 150°C,
caractérisé par le fait que, d'une part, la teneur en eau de l'acide sulfurique reste toujours inférieure à 4 % pendant la réaction et que, d'autre part, on ajoute au cours de la réaction, directement ou sous forme d'acide sulfurique dilue, au moins la quantité d'eau stoechiométriquement nécessaire à la formation d'acide quadratique.

2. Procédé selon la revendication 1,
caractérisé par le fait que pendant la réaction, il existe une solution aqueuse d'acide sulfurique dont la teneur en eau reste toujours en dessous de 3,5 %, de préférence en dessous de 2 %.

3. Procédé selon les revendications 1 et 2,
caractérisé par le fait que l'on part d'un acide sulfurique anhydre et que l'on règle la teneur en eau, après la fin du dégagement de chlorure d'hydrogéne, à une valeur de 0,05 à 3,9 % en poids.

4. Procédé selon les revendications 1 à 3, caractérisé par le fait que l'on utilise un mélange d'hexachloro-cyclobutène et d'hexachloro-butadiène, qui contient 55 et moins d'hexachloro-cyc'obutène.